# FASCICULE DE BREVET EUROPEEN

(11) **EP 2 506 856 B1**
(45) Date de publication et mention de la délivrance du brevet: **02.01.2019**
(21) Numéro de dépôt: 10799097.0
(22) Date de dépôt: 23.11.2010
(51) Int. Cl.: A61K 31/575, A61P 31/04, A61K 9/00, A61K 9/06

(54) **COMPOSÉS AMINOSTÉROÏDIENS POUR UNE APPLICATION TOPIQUE LOCALE POUR LA DÉCOLONISATION CUTANÉO-MUQUEUSE DE STAPHYLOCOCCUS AUREUS**
AMINOSTEROID VERBINDUNGEN ZUR LOKALEN TOPISCHEN APPLIKATION FÜR HAUT-SCHLEIMHAUT DEKOLONISIERUNG VON STAPHYLOCOCCUS AUREUS
AMINOSTEROID COMPOUNDS FOR TOPIC LOCAL APPLICATION FOR SKIN-MUCOSA DECOLONISATION OF STAPHYLOCOCCUS AUREUS

(30) Priorité: 02.12.2009 FR 0958575
(43) Date de publication de la demande: 10.10.2012
(73) Titulaire: Assistance Publique Hôpitaux de Marseille, 13005 Marseille (FR)
(72) Inventeur: BRUNEL, Jean-Michel, 13012 Marseille (FR); RAOULT, Didier, 13008 Marseille (FR); ROLAIN, Jean-Marc, 13006 Marseille (FR)
(74) Mandataire: Domange, Maxime
(86) Numéro de dépôt international: PCT/FR2010/052492
(87) Numéro de publication internationale: WO 2011/067501

(56) Documents cités:
- WO-A2-2008/031113
- ALHANOUT KAMEL ET AL: "In vitro antibacterial activity of aminosterols against multidrug-resistant bacteria from patients with cystic fibrosis.", THE JOURNAL OF ANTIMICROBIAL CHEMOTHERAPY OCT 2009, vol. 64, no. 4, octobre 2009 (2009-10), pages 810-814, XP002572978, ISSN: 1460-2091 cité dans la demande
- SALMI C ET AL: "Efficient preparation of secondary aminoalcohols through a Ti(IV) reductive amination procedure. Application to the synthesis and antibacterial evaluation of new 3beta-N-[hydroxyalkyl]aminosteroid derivatives", TETRAHEDRON, ELSEVIER SCIENCE PUBLISHERS, AMSTERDAM, NL, vol. 64, no. 19, 5 mai 2008 (2008-05-05), pages 4453-4459, XP022589433, ISSN: 0040-4020 [extrait le 2008-02-21] cité dans la demande
- LONCLE ET AL: "Synthesis of new 7-aminosterol squalamine analogues with high antimicrobial activities through a stereoselective titanium reductive amination reaction", TETRAHEDRON, ELSEVIER SCIENCE PUBLISHERS, AMSTERDAM, NL, vol. 63, no. 52, 19 novembre 2007 (2007-11-19), pages 12968-12974, XP022350762, ISSN: 0040-4020 cité dans la demande
- DATABASE CA [Online] CHEMICAL ABSTRACTS SERVICE, COLUMBUS, OHIO, US; 1961, HIRAMI, YOSHIHIKO: "Steroids having biological activity. II. Syntheses of the antibacterial amino steroids derived from pregnenolone", XP002572979, extrait de STN Database accession no. 1964:61131
- DEVKOTA K P ET AL: "Bioactive 5[alpha]-pregnane-type steroidal alkaloids from Sarcococca hookeriana", JOURNAL OF NATURAL PRODUCTS 200808 US, vol. 71, no. 8, août 2008 (2008-08), pages 1481-1484, XP002573052, ISSN: 0163-3864
- K. ALHANOUT ET AL: "New insights into the antibacterial mechanism of action of squalamine", JOURNAL OF ANTIMICROBIAL CHEMOTHERAPY., vol. 65, no. 8, 15 June 2010 (2010-06-15), pages 1688-1693, XP055461427, GB ISSN: 0305-7453, DOI: 10.1093/jac/dkq213

## Description

La présente invention concerne de nouveaux composés aminostéroïdiens antibactériens de type polyamino cholestane ou cholestène et l'utilisation de ces dérivés aminostéroïdiens antibactériens de type polyamino cholestane ou cholestène pour une application par voie topique locale pour la décolonisation cutanéo-muqueuse rapide de Staphylococcus aureus, notamment sous forme de pommade ou de crème.

Les infections à staphylocoques dorés sont souvent transmises par des porteurs chroniques asymptomatiques. Ces porteurs chroniques ont des réservoirs cutanés ou muqueux, en particulier la muqueuse nasale. La décontamination de la muqueuse nasale est devenue un préalable de plus en plus commun et important dans les hospitalisations car la colonisation du nez par les staphylocoques dorés est associée à un risque accru d'augmentation des infections hospitalières, en particulier vasculaires et ostéoarticulaires, notamment en cas d'opération chirurgicale sur le patient porteur de cette colonisation nasale qui peut s'auto-infecter par contamination per opératoire. Plusieurs types de produits ont été utilisés dans le passé pour tenter d'éradiquer les staphylocoques présents dans les narines, en particulier des antibiotiques. Des produits contenant de la rifampicine puis de la fucidine ont été utilisés mais très rapidement les staphylocoques sont devenus résistants à ces antibiotiques pour lesquels des mutants sont facilement sélectionnés. Depuis une vingtaine d'années, le produit le plus utilisé est une pommade à la mupirocine, qui est un produit à activité antibiotique, qui n'est utilisée dans aucune autre indication. Le fait que ce produit ne soit pas utilisé dans d'autres indications ménage l'écologie de la résistance des staphylocoques pour les infections générales par voie systémique (sanguine). L'augmentation de la résistance à la mupirocine ne risquait pas d'avoir des conséquences sur la prise en charge thérapeutique des infections systémiques à staphylocoque doré avec d'autres antibiotiques. Par ailleurs, l'absence de prescription de mupirocine dans d'autres circonstances que l'éradication des staphylocoques dans les fosses nasales a évité une prescription massive de ce produit et a donc retardé l'apparition de mutants résistants. La mupirocine permet de diviser la concentration de bactéries Staphylococcus sur un modèle de colonisation in vitro de 5.10² (CFU/ml) au bout de 24 heures (référence 18). Les résultats in vivo étant moins favorables, en pratique, pour la mupirocine, on applique une pommade contenant le composé à une concentration de 1%, 2 fois/jour, matin et soir, pendant 48 heures, avant une opération chirurgicale, pour obtenir une décolonisation de 99,9% des bactéries *Staphylococcus aureus,* notamment dans les fosses nasales, et on poursuit le traitement pendant 3 jours, après l'opération chirurgicale. Malheureusement, apparaissent maintenant des mutants résistants à la mupirocine qui risquent de s'étendre extrêmement rapidement du fait d'une part de la capacité de transfert latéral de cette résistance entre les souches et d'autre part de l'usage intensif de la mupirocine actuellement pour la prévention des infections staphylococciques. Récemment, l'utilisation de lysostaphine s'est avérée très efficace (comparativement à la mupirocine) pour la décolonisation nasale mais elle présente également une limitation d'emploi puisque des souches résistantes sont apparues.

En 1993, la squalamine (Formule Ia), un stéroïde naturel, isolée majoritairement des tissus d'un petit requin Squalus acanthias, s'est révélée être une substance très active présentant essentiellement une activité antiangiogénique contre les cellules et une activité anti virale et antibactérienne.

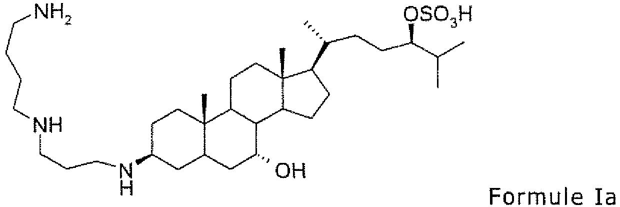

Chimiquement, la squalamine Ia est une molécule originale présentant un caractère amphiphile. Elle comporte ainsi, une partie centrale apolaire (un squelette de type cholestane) et deux extrémités polaires (une chaîne polyamine et un groupement sulfate).

Initialement, ce polyaminostérol hydrosoluble avait suscité l'intérêt pour ses propriétés antiangiogéniques et antimicrobiennes sur une variété de bactéries à Gram positif (*Staphylococcus aureus, Enterococcus faecalis*) et à Gram négatif (*Escherichia coli, Pseudomonas aeruginosa*), des champignons (*Candia albicans, Candida tropicalis*) et des protozoaires.

La source naturelle de la squalamine étant limitée, on a recherché des dérivés synthétiques analogues aminostéroïdiens de la squalamine. On a décrit notamment des dérivés ou analogues comportant une chaine polyaminée en position 3 ou 7 de cycles 10, 13 diméthyl, 17 octane cholestane ou cholestène, éventuellement hydroxylés en position 7 ou respectivement 3. En particulier, des dérivés ont été décrits comme présentant une activité antibactérienne similaire à la squalamine vis-à-vis de diverses bactéries Gram positif et Gram négatif multirésistantes (références 6 et 9 à 14). On a plus particulièrement suggéré une application de ces dérivés pour un traitement curatif des infections pulmonaires par voie aérosol. Toutefois les Concentrations Minimales Inhibitrices (CMI) de la squalamine et de ces composés aminostéroïdiens contre ces microorganismes sont trop élevées pour pouvoir envisager une utilisation par voie générale ou par voie aérosol sans toxicité.

C'est pour cette raison que ces composés aminostéroïdiens n'ont été envisagés à ce jour qu'à titre d'agent cytotoxique anti-angiogénique pour le traitement de tumeurs (référence 17).

En outre, dans les différentes publications antérieures (références 9 à 14), on n'avait pas élucidé le mécanisme d'action de l'activité antibactérienne in vitro de la squalamine et ses dérivés synthétiques analogues aminostéroïdiens et, surtout, on n'avait pas observé que ces composés n'induisaient pas de résistance à la bactérie *Staphylococcus aureus* et n'induisaient, non plus, de toxicité cutanéo-muqueuse par application topique locale.

Le but de la présente invention est de fournir des nouvelles molécules anti *Staphylococcus aureus* aptes à être appliquées par voie topique pour une action locale de décolonisation cutanéo-muqueuse de la bactérie *Staphylococcus aureus,* notamment dans le cadre d'un traitement prophylactique d'une infection postopératoire opportuniste dans un organe du patient accessible pendant l'opération chirurgicale, telle que la muqueuse nasale, et ce sans induire de toxicité cutanéo-muqueuse.

On entend ici par "décolonisation" cutanéo-muqueuse, qu'il ne s'agit pas du traitement curatif d'une infection au *Staphylococcus aureus* chez un patient, mais d'une éradication au moins transitoire de la bactérie présente sur la peau ou une muqueuse corporelle de manière asymptomatique chez un porteur sain.

Un autre but de la présente invention est de fournir des molécules anti *Staphylococcus aureus* non utilisées par voie générale ou systémique, n'induisant pas de résistance de la bactérie *Staphylococcus aureus* et ayant une activité de décolonisation supérieure et/ou plus rapide à celle d'autres composés connus utilisés dans la décolonisation cutanéo-muqueuse de la bactérie *Staphylococcus aureus,* notamment par rapport à la mupirocine.

Pour ce faire la présente invention fournit un composé aminostéroïdien analogue de la squalamine tel que défini dans l'une des revendications 1 à 5 ainsi qu'une composition pour une utilisation dans une méthode de traitement thérapeutique telle que définie dans l'une des revendications 6 à 15, notamment une composition pour une utilisation dans une méthode de traitement thérapeutique consistant dans une décolonisation cutanéo-muqueuse de *Staphylococcus aureus,* ledit composé aminostéroidien analogue de la squalamine répondant à une formule générale comprenant un squelette de formule (I) ci-après sur lequel sont greffées 2 chaînes -NHR, R étant une chaine hydrocarbonée éventuellement substituée comportant au moins un groupe -NH₂ tel que décrit ci-après: 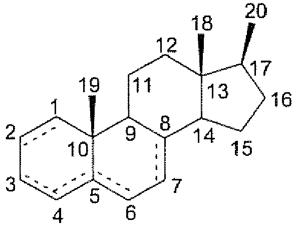 dans laquelle:
- soit toutes les liaisons doublées de traits en pointillés entre les carbones des positions 7-8, 5-6, 4-5, et 3-4 représentent une simple liaison, le squelette étant celui d'un 10, 13, 17 triméthyl cholestane,
- soit l'une des liaisons doublées de traits en pointillés entre les carbones des positions 7-8, 5-6, 4-5, et 3-4 représente une double liaison et les autres liaisons doublées de traits en pointillés sont des simples liaisons, le squelette étant celui d'un 10, 13, 17 triméthyl cholestène.

Selon la présente invention, ledit nouveau composé répond à la formule générale constituée par un dit squelette de formule (I) comportant :
a) 2 chaines identiques -NHR sur les carbones en positions 3 et, respectivement, 20, et R représente -[(CH₂)n-(NR₁)ₖ-(CH₂)ₘ]ₚ-NH₂
   avec :
   - n et m étant des entiers identiques ou différents de 1 à 7,
   - k= 0 ou 1,
   - p étant un nombre entier de 1 à 4,
   - R₁ étant choisi parmi H, un alkyl en C1 à C3, un phényl et un groupe -COOalk, alk étant un alkyl en C1 à C3, R1 étant éventuellement substitué par OH, NH₂, SH, ou OSO₃H,
   avec, de préférence, lorsque k= 0, alors p=1, ou lorsque k= 1, alors p= 2, et
b) les carbones aux autres positions du dit squelette de formule (I) comprenant un radical R₀ identique ou différent choisi parmi H, NH₂,SH, ou R₁, de préférence R₀ étant H ou OH mais avec 1 seul R₀ étant OH.

On entend ici par application topique locale, une procédure qui consiste à appliquer le composé sur la peau ou une muqueuse pour obtenir un effet local en traversant le derme ou la muqueuse pour être absorbé exclusivement par le derme ou la muqueuse, ledit composé étant formulé en lotion, crème, onguent, pâte ou pommade, à l'exclusion donc des aérosols , dans le cas d'une application par voie nasale ou buccale, pour éviter toute absorption pulmonaire.

De préférence, l'utilisation selon l'invention concerne une application sur la muqueuse nasale. Toutefois, l'utilisation envisagée ne se limite pas à une application sur la muqueuse nasale mais également à différents endroits sur la peau, notamment au niveau des plis cutanés et, plus particulièrement, au niveau des aisselles.

Dans un mode préféré de réalisation, lesdits composés selon l'invention sont utilisés pour un traitement préventif ou prophylactique préopératoire visant à prévenir une infection postopératoire à *Staphylococcus aureus* chez un patient porteur sain auquel on va faire subir une opération chirurgicale.

Selon la présente invention, on a découvert et démontré que la squalamine et ses dérivés synthétiques analogues aminostéroïdiens n'induisent pas de résistance pour la bactérie *Staphylococcus aureus*. Cette propriété est corroborée par le fait, également démontré selon la présente invention, que l'activité bactéricide de la squalamine et ses dérivés synthétiques analogues aminostéroïdiens sur *Staphylococcus aureus* relève d'un mécanisme d'action physicochimique de type détergent faisant éclater et détruisant la paroi de la bactérie et que ces composés n'agissent donc pas comme des antibiotiques. On sait, en effet, que les antibiotiques agissent par inhibition d'un processus essentiel à la multiplication bactérienne codé par une cible génomique en bloquant soit la réplication du génome soit en inhibant la synthèse d'un facteur protéique tel qu'une enzyme nécessaire à la survie et/ou croissance de la bactérie, notamment la synthèse de la paroi. Les bactéries peuvent résister à ces mécanismes d'action des antibiotiques par le biais de mutations géniques induisant l'apparition d'une résistance à l'antibiotique en cause. De telles mutations conférant une résistance sont en revanche improbables face au mécanisme d'action bactéricide direct par destruction physico-chimique de la paroi bactérienne. A cause de ce mécanisme d'action bactéricide de la squalamine et ses dérivés synthétiques analogues aminostéroïdiens selon l'invention, leur activité reste conservée même s'il s'agit uniquement d'un portage cutanéo-muqueux de la bactérie, sans induire de résistance à ces composés de la part de la bactérie *Staphylococcus*.

D'autre part, les composés de squalamine et dérivés aminostéroïdiens présentent une nature amphiphile. En effet, chimiquement, ils comportent une partie centrale apolaire (un squelette de type cholestane ou cholestène) et une extrémité polaire (une chaîne polyamine et un groupement hydroxyl). Il en résulte qu'ils sont susceptibles d'être incorporés dans un excipient lipophile, tout en étant capables d'en être libérés progressivement et d'interagir avec la peau et une muqueuse de par leur composante hydrophile, mais cette action restant locale, c'est-à-dire sans absorption du composé dans l'organisme par voie percutanée et évitant ainsi d'induire une toxicité.

Ainsi, il a été démontré que ces composés présentent une activité in vivo de décolonisation cutanée d'une colonisation à *S*. *aureus* en application topique locale sur la souris et ce sans induire de toxicité par lésions cutanées ni mortalité subséquente de la souris. De plus, on a démontré à l'aide d'essais comparatifs que d'autres composés connus comme ayant une activité de décolonisation anti-Staphylococcus aureus (acide fusidique, vancomycine et mupirocine) présentaient une activité nettement moindre et moins rapide que celle de la squalamine et analogues de la squalamine. Certains résultats des essais in vitro et in vivo sur la souris sont rapportés dans la description détaillée ci- après.

Plus particulièrement, les composés analogues de squalamine selon l'invention sont utiles pour la décolonisation cutanéo-muqueuse de Staphylococcus aureus d'au moins 99,90% des bactéries *Staphylococcus* aureus (à savoir une division d'au moins 10³ de la concentration bactérienne), de préférence d'au moins 99,99% (à savoir une division d'au moins 10⁴ de la concentration bactérienne), pas plus de 24 heures, de préférence pas plus de 1 heure, après seulement une seule application, cette décolonisation étant maintenue, de préférence encore, pendant au moins 48 heures. Les composés analogues de squalamine selon l'invention ont donc une activité supérieure et plus rapide de décolonisation par rapport à d'autres composés connus, comme la mupirocine, sur un modèle de colonisation cutané chez la souris après une seule application sous forme de pommade.

En l'espèce, selon l'invention, on obtient une activité supérieure et plus rapide à celle d'autres composés connus en ce que l'on réduit une concentration initiale de bactéries *Staphylococcus aureus* de plus de 10⁴ CFU/ml, sur un modèle de colonisation cutané chez la souris, à moins de 10¹ CFU/ml en moins de 24 heures, plus précisément encore, à moins de 1 CFU/ml en moins de 1 heure, après une seule application d'une pommade contenant les composés selon l'invention à une concentration de 1%.

En pratique, les colonisations cutanéo-muqueuses de Staphylococcus aureus, notamment dans les fosses nasales, n'excèdent jamais 10⁵ CFU/ml et, plus généralement, n'excèdent jamais 10⁴ CFU/ml.

En pratique, les composés selon l'invention présentent une activité bactéricide in vitro sur *Staphylococcus aureus,* c'est-à-dire qu'on est capable de réduire d'au moins 99.9% un inoculum bactérien initial de 10⁶ CFU/ml incubé pendant 24 heures aux concentrations utilisées (Référence 15 : Daniel Amsterdam. Susceptibility testing of antimicrobials in liquid media. In Antibiotics in Laboratory Medicine. 5th Edition. Editor Victor Lorian. Lippincott Williams and Wilkins 2005. Philadelphia, USA), et la concentration de bactéries est réduite à moins de 10² CFU/ml au bout de 48 heures après une seule application de pommade au temps t=0.

Ces composés analogues de la squalamine présentent une activité antibactérienne in vitro avec notamment une CMI sur *Staphylococcus aureus* inférieure à 50 µg/ml, de préférence inférieure à 15 µg/ml.

Les composés selon l'invention les plus actifs, de formules IIIa, IIIb, III-1 ci-après, présentent des CMI sur *Staphylococcus aureus* inférieures à 5 µg/ml.

Ces composés peuvent être préparés par les procédés décrits dans les articles des références 9 à 14, notamment 9 et 13, listés en fin de description, consistant essentiellement à réaliser une réaction d'amination réductrice au titane entre les différentes cétones (cholestenone, cholestanone) et diamines mises en oeuvre.

Plus particulièrement, lesdits nouveaux composés répondent aux formules générales IIIa, IIIb et III-1 suivantes, dans lesquelles:
- R représente -[(CH₂)ₙ-(NR₁)ₖ-(CH₂)ₘ]ₚ-NH₂, m, n, p, k et R₁ ayant les significations données ci-dessus, et, de préférence, R étant choisi parmi -(CH₂)ₙ₁-NH₂ avec n₁= 2 à 14, et -(CH₂)₃-NH-(CH₂)₃-NH-(CH₂)₃-NH₂:

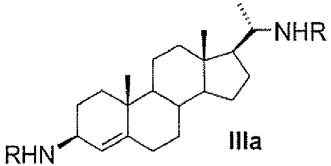

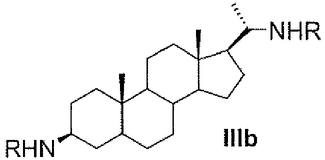

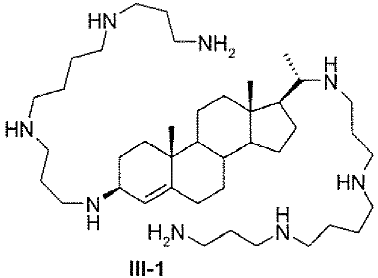

Les nouveaux composés selon l'invention ci-dessus peuvent être préparés simplement par amination réductrice au titane entre un produit de départ correspondant au produit final mais avec une fonction carbonyl ou cétone -CO- en lieu et place de la fonction -NHR- et une diamine NH2R via la formation transitoire des imines correspondantes réduites in situ par le borohydrure de sodium.

Plus particulièrement pour les composés de formules IIIa et IIIb, la synthèse de ces dérivés met en jeu une réaction d'amination réductrice au titane entre la progestérone IVa ou respectivement la progestanone IVb et une diamine via la formation transitoire des 3,20 diimino polyamino cholestanes ou cholestènes avec un radical de formule =NR en positions 3 et 20 -correspondantes réduites ensuite in situ par un borohydrure de sodium.

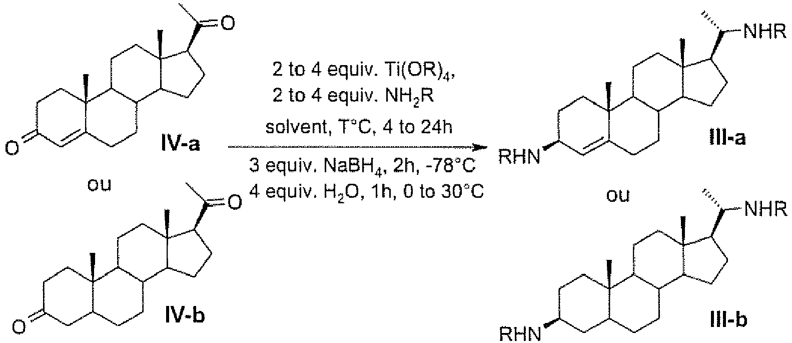

Différentes sources de titane de type Ti(OR')₄ avec R'= Me, Et, Propyl, Isopropyl, butyl sont facilement disponibles commercialement.

Différents solvants de réaction CH₂Cl₂, CHCl₃, THF, Ethanol, Isopropanol sont appropriés.

Le contrôle de la stéréosélectivité au niveau du carbone portant la chaine aminée se fait en fonction de la nature de l'agent réducteur employé en cours de procédé. Ainsi la réduction réalisée avec le borohydrure de sodium (NaBH₄) conduit majoritairement aux dérivés béta tandis que l'emploi de cyanoborohydrure de sodium (NaBH₃CN) ou bien d'oxyde de platine/hydrogène conduit à la formation préférentielle des dérivés alpha.

Sur ce principe général de synthèse, de nombreux dérivés 3β-20β polyamino-4-cholestenes de formule IIIa et 3β-20β polyamino cholestanes de formule IIIb ont été préparés avec -NHR choisi parmi :

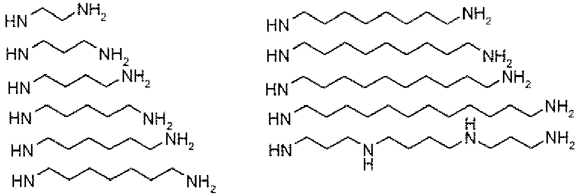

La présente invention fournit également une composition pharmaceutique utile pour une utilisation par voie topique locale pour une décolonisation cutanéo-muqueuse de *Staphylococcus aureus* caractérisée en ce qu'elle comporte, comme composé actif, un dit composé aminostéroidien analogue de la squalamine antibactérien selon l'invention incorporé dans un excipient lipophile sous forme de lotion, crème, pommade ou onguent.

Ainsi, les composés selon l'invention sont capables d'être libérés progressivement et d'agir avec la peau et une muqueuse, cette action restant locale, c'est-à-dire sans absorption du composé dans l'organisme par voie percutanée et évitant ainsi d'induire une toxicité.

Selon d'autres caractéristiques particulières préférées de la composition selon l'invention :
- la concentration de dit composé aminostéroidien analogue de la squalamine est de 0,5 à 5%, de préférence au moins 1%, plus particulièrement de 1 à 2%, en poids,
- ledit excipient lipophile est choisi parmi la vaseline, lanoline, les esters de glycérol, esters d'acides gras et de macrogols (PEG). Ces excipients lipophiles sont avantageux car ils ne s'absorbent pas par voie per cutanée,
- ledit composé aminostéroidien analogue de la squalamine antibactérien est sous forme de sel hydrosoluble, de préférence "sous la forme de sel de chlorhydrate, bromhydrate, triflate, phosphate, lactate, ou succinate"

Ces deux dernières caractéristiques permettent d'optimiser la valeur de la concentration et donc l'action topique locale desdits composés actifs.

D'autres caractéristiques de la présente invention apparaitront à la lumière de la description détaillée suivante faite en référence aux figures 1 à 6 suivantes dans lesquelles:
- la figure 1 représente une photo du mécanisme de destruction de la paroi bactérienne sur une souche de *S. aureus,*
- la figure 2 représente le graphique de réduction de concentration bactérienne d'une souche de *S. aureus* au cours du temps t par un traitement par les composés squalamine la (-□-), composé II-1 (-Δ-) et contrôle (-0-),
- la figure 3 représente des photographies du modèle expérimental d'infection à *S. aureus* chez la souris à J=0 et des colonies à J1 (1 jour après infection) et J2 (2 jours après infection) sur gélose Chapman,
- la figure 4 représente un histogramme des dénombrements des colonies de *S. aureus* isolées à partir du modèle expérimental de colonisation cutanée chez la souris après application de pommade avec la squalamine la (- 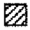 - à droite) ou sans molécule active (- 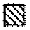 - à gauche) sur gélose Chapman, en fonction du temps, à t=0, t=1 jour et t=2 jours,
- la figure 5 représente les photographies des colonies de *S*. *aureus* isolées à partir du modèle expérimental de colonisation cutanée chez la souris après application de pommade avec un dérivé aminostéroïdien II-1 (A) ou sans molécule active (B) sur gélose Chapman, obtenues après 1 jour d'application de pommade,
- la figure 6 représente le graphique des dénombrements des colonies de *S. aureus* isolées à partir du modèle expérimental de colonisation cutanée chez la souris, après une seule application de la pommade avec la squalamine la (-□-), le composé II-1 (-O-) ou avec l'acide fusidique (-Δ-) ou avec la vancomycine (-∇-) ou avec la mupirocine (-◊-) sur gélose Chapman, en fonction du temps, après 30 minutes et 1 heure.

### Exemple comparatif 1: préparation du composé II-1 (non couvert par l'invention)

Dans un ballon bicol mis sous argon, 3 équivalents de butane diamine (0,69 10⁻³ mol, 61 mg) sont dissous dans 5 ml de MeOH, puis on additionne 87 µl de Ti(Oipr)₄ (0,30 10⁻³ mol). Après 2 minutes d'agitation, on ajoute au mélange 100 mg de 7-cétocholest-5-ene-3β-ol (0,23 10⁻³ mol). Après 24 heures sous agitation, on place le ballon à -78°C, puis 11 mg de NaBH₄ (0,23 10⁻³ mol) sont ajoutés. La phase organique est séchée sur Na₂SO₄, filtrée puis évaporée sous vide poussé. On purifie le produit par une chromatographie sur gel de silice (éluant: CH₂Cl₂/MeOH/NH₄OH (7/3/1)).

7β-(1,4-diaminobutane)-cholest-5-ene-3β-ol II-1: Purification par chromatographie sur gel de silice (silicagel; CH₂Cl₂/MeOH/NH₄OH (32%), 7:3:1). Solide jaune clair (Rdt: 70%); -
¹H NMR (300 MHz, CDCl₃): δ= 5.27-5.64 (m, 1H), 0.63-3.49 (m, 55H); - ¹³C NMR (75 MHz, CDCl₃): δ = 141.75, 123.99, 70.80, 60.32, 56.79, 55.72, 55.22, 53.02, 49.46, 48.32, 43.21, 42.07, 39.37, 38.98, 37.28, 36.09, 35.90, 35.67, 35.59, 27.88, 26.11, 24.44, 23.71, 22.70, 22.44, 20.70, 18.96, 18.65, 18.59, 11.96, 11.50. C₃1H₅₆N₂O₁; MS (ESI) m/z = 473.5 [M+H]⁺.

### Exemple 2 : préparation du composé III-1

Dans un ballon bicol mis sous argon, 140 mg de spermine H₂N-(CH₂)₃-NH-(CH₂)₄-NH-(CH₂)₃-NH₂ sont additionnés à 200 µl de Ti(Oipr)₄ (0,70 10⁻³ mol). Après 2 minutes d'agitation, on ajoute au mélange 100 mg de progestérone IVa (0,31 10⁻³ mol). Après 24 heures sous agitation, on place le ballon à -78°C, puis 37 mg de NaBH₄ (0,70 10⁻³ mol) sont ajoutés. 2 heures plus tard, 2 ml d'eau sont ajoutés pour arrêter la réaction. 5 minutes après, le mélange est filtré sur frité et célite. La phase organique est séchée sur Na₂SO₄, filtrée puis évaporée sous vide poussé. On purifie le produit par une chromatographie sur gel de silice (éluant: CH₂Cl₂/MeOH/NH₄OH (7/3/1)).

Purification par chromatographie (gel de silice ; dichlorométhane, méthanol, ammoniaque, 70/30/10); Solide jaune clair (Rdt: 58%)- RMN ¹H (300 MHz, CD₃OD): δ= 5.30 (s, 1H), 3.30-2.67 (m, 23H), 2.77-1.02 (m, 55H), 0.80-0.66 (m, 6H).- RMN ¹³C (75 MHz, CD₃OD): δ= 148.69, 122.37, 71.21, 69.24, 65.10, 61.68, 59.80, 58.00, 57.57, 56.48, 56.41, 56.23, 54.10, 46.48, 45.79, 45.63, 44.09, 43.81, 40.87, 40.41, 39.08, 37.93, 37.70, 34.75, 33.88, 32.44, 31.61, 30.60, 30.05, 29.68, 28.35, 27.26, 25.88, 24.20, 22.75, 22.43, 19.97. C₄₃H₈₅N₇.

### Exemple 3 : mécanisme d'action bactéricide des composés la, II-1 et III-1 et résistance de Staphylococcus aux dits composés.

On a étudié le mécanisme d'action de ces composés par microscopie électronique afin de savoir si une éventuelle résistance à ces composés était prévisible au cours du temps. Pour ce faire, on a mis à incuber une souche de référence de *S*. *aureus* (ATCC 25923) cultivée en milieu liquide en présence de respectivement la squalamine de formule la (non couvert par l'invention) ou du dérivé II-1 (non couvert par l'invention) ou dérivé III-1 (selon l'invention) aux mêmes concentrations de 8 µg/ml pendant 24 heures. Puis, on a fixé les bactéries ainsi incubées avec du glutaraldéhyde à 2.5%. Après fixation au tétroxyde d'osmium pendant 1 heure et déshydratation par l'éthanol, les échantillons ont été inclus dans de la paraffine (Epon 812 resin). Des coupes de 70 nm ont ensuite été réalisées puis marquées avec de l'acétate d'uranyle à 4% puis visualisées à l'aide d'un microscope électronique à transmission de type Philips Morgavi 268D.

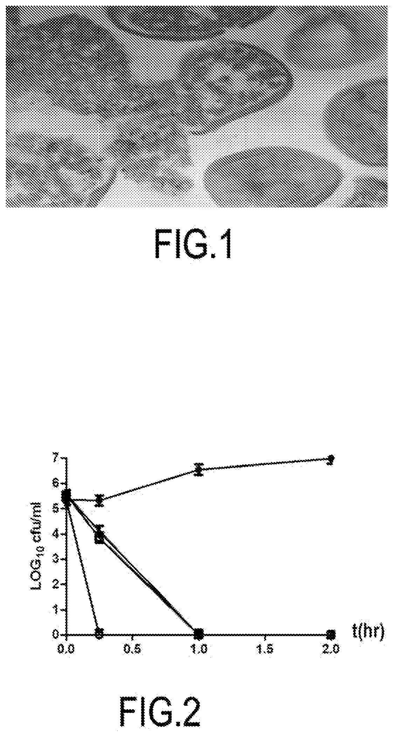

On a pu montrer que l'action de ces composés sur *S. aureus* était nouvelle et originale avec un effet physico-chimique spectaculaire par destruction de la paroi microbienne, comme représenté figure 1, et non par un mécanisme d'action de type antibiotique. Cet effet est par ailleurs quasiment immédiat comme le montre l'analyse in vitro réalisée au cours du temps représenté (figure 2).

Du fait de cet effet physico-chimique létal irréversible rapide, on comprend pourquoi il n'a pas été possible de sélectionner des mutants résistants in vitro contre ces composés au cours du temps. A ce jour aucune des souches de *S. aureus* testées n'a présenté de résistance aux différents dérivés aminostéroïdiens connus et nouveaux aminostéroïdiens selon la présente invention. Ceci a été réalisé selon une méthode de référence (Banerjee R et al. In Vitro Selection and Characterization of Ceftobiprole-Resistant Methicillin-Resistant Staphylococcus aureus. Antimicrob Agents Chemother 2008, 52(6) :2089-2096) en essayant de sélectionner in vitro des souches de *S. aureus* (ATCC 25923) résistantes aux dits composés. Les composés ont été dilués successivement (dilutions de raison 2) dans des tubes contenant 5 ml de milieu MH afin d'obtenir des concentrations variant de 0.5X la CMI à 4X la CMI. La suspension bactérienne est ajoutée dans chaque tube à une densité de 10⁹ CFU/ml. Après incubation de 18 h à 37 °C, le dernier tube à croissance visible (0,5 x CMI) dilué au 1/20 est mis en présence d'une nouvelle série de dilutions géométriques équivalentes à celles du 1er jour pour le même composé. Les essais ont été répétés 10 fois en triplicata mais n'ont jamais permis d'isoler de bactéries en croissance dans les tubes contenant les dérivés aminostéroïdiens à des concentrations égales ou supérieures à leur CMI. On n'a donc pas réussi à obtenir de mutants résistants à ces molécules.

Ce résultat est à rapprocher de celui obtenu avec des antiseptiques à base d'alcool ou d'iode qui sont utilisés comme antiseptiques locaux (peau et muqueuses) depuis des dizaines d'années sans qu'aucune résistance ne soit apparue. Ceci est totalement différent pour la rifampicine, l'acide fusidique ou la mupirocine pour lesquels des résistances apparaissent très fréquemment et très rapidement. En conclusion on a montré que compte tenu de l'effet physicochimique de ces composés et de l'absence d'obtention de mutants résistants in vitro, l'éventualité d'un développement futur de résistance à ces composés est improbable.

Ce nouveau mécanisme d'action de type létal, irréversible et quasiment immédiat sur la paroi bactérienne des composés selon l'invention rend improbable le développement de résistances dans le futur.

### Exemple 4 : activité bactéricide in vitro et mesure de CMI

On a rapporté dans la publication de Alhanout et al (6) que les CMI de la squalamine et du dérivé II-1 sur différentes souches de *S*. *aureus* (y compris des souches résistantes à la méticilline (MRSA)) étaient égales (2µg/ml).

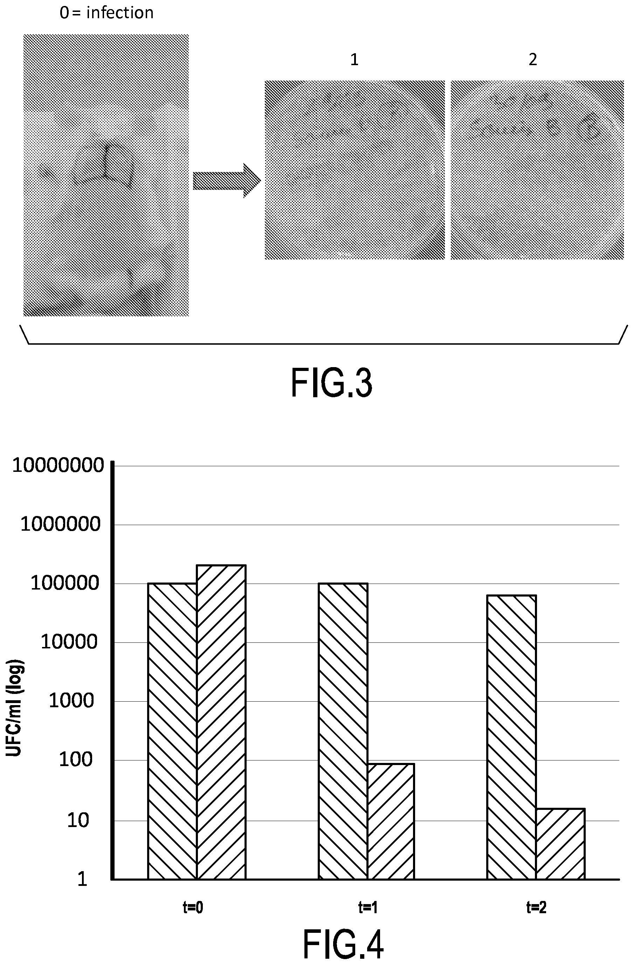

Les nouveaux composés de la présente invention de formules IIIa, IIIb et III-1 présentent une meilleure activité in vitro contre les souches de *S. aureus* et dérivé II-1 présentant une CMI de 1 µg/ml et une activité bactéricide beaucoup plus rapide (15 minutes, figure 3, dérivé III-1).

Les composés IIIa et IIIb (selon l'invention) avec
R=-(CH₂)ₙ₁-NH_{Z} et n₁=2 à 9 présentent une CMI de 2,5 à 20 µg/ml.

**Tableau 1 : CMI des dérivés III-a sur S. aureus ATCC 25923**

| n₁ | CMI (µg/mL) |
|---|---|
| 2 | 20 |
| 3 | 5 |
| 4 | 5 |
| 5 | 5 |
| 6 | 2,5 |
| 7 | 2,5 |
| 8 | 2,5 |
| 9 | 5 |

Le composé III-1 présente la meilleure activité avec une CMI de 1µg/ml pour *S. Aureus.*

**Tableau 2 : CMI de la squalamine Ia et des dérivés II-1 et III-1 sur différentes souches de S. aureus.**

| Reference strain | CMI mg/l | | |
|---|---|---|---|
| | Ia | Dérivé II-1 | Dérivé III-1 |
| *Staphylococcus aureus* ATCC 25923 | 2 | 2 | 1 |
| *Staphylococcus aureus* (MRSA) | 2 | 2 | 1 |

SQ: squalamine

En conclusion, on a démontré que le dérivé III-1 synthétisé spécifiquement pour cette application présente la meilleure activité in vitro ainsi qu'un effet bactéricide plus rapide par rapport aux autres composés évalués in vitro.

### Exemple 5 : modèle de test d'activité in vivo sur la souris

Afin de tester l'activité in vivo des composés, on a inventé un modèle expérimental de colonisation cutanéo-muqueuse à *S. aureus* chez la souris. En effet, les modèles expérimentaux existants sont compliqués, fastidieux et/ou très coûteux à mettre en oeuvre (colonisation nasale chez le rat ou modèle ex vivo sur de la peau humaine) (7;8). Brièvement, 10 µl d'une suspension bactérienne calibrée à 10⁸ CFU/ml *S. aureus* a été appliquée sur la peau préalablement rasée des souris sur deux quadrants différents (figure 3). La colonisation par *S. aureus* était ensuite suivie quotidiennement pendant 2 jours par écouvillonnage cutané des souris, reprise de l'écouvillon dans 1 ml d'eau stérile et mise en culture de 100 µl sur gélose Chapman à 37°C pendant 24 heures avant énumération des colonies bactériennes. Le nombre de CFU/ml était stable pendant le temps de la manipulation soit 2 jours (figure 4).

Sur la figure 3, on a représenté des photographies du modèle expérimental d'infection à *S. aureus* chez la souris; et des colonies à J1 et J2 sur gélose Chapman.

Ce modèle expérimental de colonisation cutanéo-muqueuse à *S*. *aureus* chez la souris s'avère être plus simple à mettre en oeuvre et permet de tester l'efficacité des composés in vivo.

### Exemple 6 : préparation d'une pommade et activité topique locale sur la souris

Une pommade à usage cutanéo-muqueux à 1% de squalamine ou de dérivés aminostéroïdiens II-1 (non couvert par l'invention) ou III-1 (selon l'invention) a été élaborée en mélangeant 9,9 g de vaseline pour 0,1 g de produit actif et évaluée dans la décolonisation cutanée de *S. aureus* (souche de référence ATCC 25923) sur le modèle de souris colonisée à *S. aureus.* De la même manière, 10 µl d'une suspension bactérienne calibrée à 10⁸ CFU/ml *S. aureus* a été appliquée sur la peau préalablement rasée d'une série de 4 souris sur deux quadrants différents (figure 3). Après 2 heures d'incubation et séchage, la pommade contenant la squalamine Ia ou un dérivé aminostéroïdien II-1 ou III-1 a été appliquée sur un des deux quadrants de peau contre une pommade contenant l'excipient seul sur l'autre quadrant. Les quantités de *S. aureus,* au temps 0 (avant application de la pommade) puis aux jours 1 et 2 (J1 et J2) après application, ont été déterminées par écouvillonnage cutané et mise en culture sur milieu Chapman pour le dénombrement des colonies comme décrit précédemment. Le nombre moyen de CFU/ml pour les souris ayant reçu la pommade contenant la squalamine la ou un dérivé aminostéroïdien II-1 ou III-1 à J1 et J2 était très significativement diminué par rapport à la pommade ne contenant pas de molécule active avec une diminution de 4 à 5 logarithmes du nombre de CFU/ml

La figure 4 représente le graphique des dénombrements des colonies de S. aureus après application de pommade avec la squalamine la ou sans molécule active sur gélose Chapman obtenus en fonction du temps. Les concentrations en bactéries *Staphylococcus aureus* données en ordonnées sont représentées en échelle logarithmique et les données de temps en abscisses sont des nombres de jours. Sur la figure 4, on passe d'environ 10⁶ CFU/ml à J0 à 10² CFU/ml à J1 et environ 10¹ CFU/ml à J2.

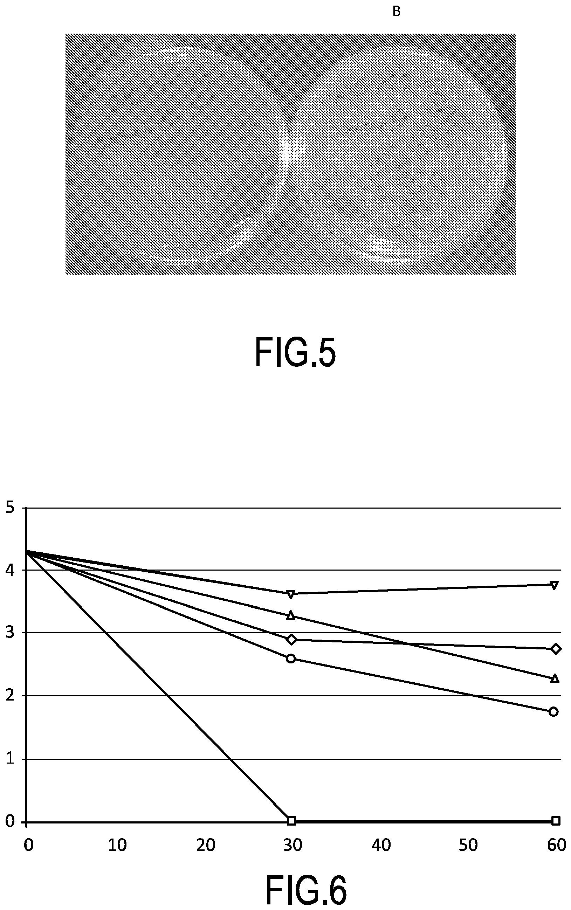

La figure 5 représente les photographies des dénombrements des colonies de *S. aureus* après application de pommade avec un dérivé aminostéroïdien II-1 (A) ou sans molécule active (B) sur gélose Chapman obtenus après 1 jour d'application de pommade.

On a montré qu'une pommade à base de dérivés aminostéroïdiens Ia, II-1 ou III-1 diminuait de manière très significative une colonisation cutanéo-muqueuse à *S. aureus* et ceci très rapidement en une seule application après 1 jour alors que la mupirocine est utilisée à raison de 2 applications par jour pendant 5 jours pour la décolonisation nasale de *S. aureus.*

### Exemple comparatif 7 : essais comparatifs avec d'autres composés connus comme ayant une activité anti-Staphylococcus aureus.

Une pommade à usage cutanéo-muqueux à 1% de squalamine ou de dérivés aminostéroïdiens II-1 a été élaborée en mélangeant 9,9 g de vaseline pour 0,1 g de produit actif et évaluée dans la décolonisation cutanée de *S. aureus* (souche de référence ATCC 25923) sur le modèle de souris colonisée à *S. aureus.* On a également utilisé à fin de comparaisons une pommade à base de mupirocine (Bactroban®), d'acide fusidique (Fucidine®) ou de vancomycine et vaseline à titre de contrôle dans les mêmes conditions expérimentales. Ainsi, 10 µl d'une suspension bactérienne calibrée à 10⁶ CFU/ml *S. aureus* a été appliquée sur la peau préalablement rasée d'une série de 6 souris sur deux quadrants différents (figure 4). Après 2 heures d'incubation et séchage, la pommade a été appliquée sur un des deux quadrants de peau contre une pommade contenant l'excipient seul sur l'autre quadrant. Les quantités de *S. aureus,* au temps 0 (avant application de la pommade) puis après 30 minutes et 1 heure d'application, ont été déterminées par écouvillonnage cutané et mise en culture sur milieu Chapman pour le dénombrement des colonies comme décrit précédemment. Le nombre moyen de CFU/ml pour les souris ayant reçu la pommade contenant la squalamine la ou un dérivé aminostéroïdien II-1 après 30 minutes et 1 heure d'application était très significativement diminué par rapport à la pommade ne contenant pas de molécule active ou bien une autre molécule de type mupirocine, vancomycine ou acide fusidique avec une diminution de 4 logarithmes du nombre de CFU/ml après 1 heure pour la pommade contenant la squalamine la (figure 6).

La figure 6 représente le graphique des dénombrements des colonies de *S. aureus* après une seule application de pommade avec la squalamine Ia ou sans molécule active ou avec un autre composé connu comme ayant une activité anti-*Staphylococcus aureus* sur gélose Chapman obtenus en fonction du temps après 30 minutes et 1 heure.

Pour le composé la, on passe d'environ 2.10⁴ CFU/ml à t=0 à une concentration inférieure à 1 CFU/ml à t=30 minutes. Et, pour le composé II-1, on passe d'une concentration d'environ 2.10⁴ CFU/ml à t=0 à une concentration de 55 CFU/ml au bout de 60 minutes. Ce résultat pour le composé II-1 permet d'escompter à tout le moins une décolonisation d'au moins 99,90% en moins de 24 heures.

En conclusion de cette expérience on peut affirmer que la squalamine et le composé II-1 sous forme de pommade présentent une activité plus rapide et nettement supérieure aux autres composés connus comme ayant une activité anti-*Staphylococcus aureus,* en particulier les pommades commercialisées pour cette indication à base de mupirocine (Bactroban®) et d'acide fusidique (Fucidine®) et ceci en seulement 1 heure et une seule application.

### Références

(1) Falagas ME, Bliziotis IA, Fragoulis KN. Oral rifampin for eradication of Staphylococcus aureus carriage from healthy and sick populations: a systematic review of the evidence from comparative trials. Am J Infect Control 2007 Mar;35(2):106-14.
(2) Howden BP, Grayson ML. Dumb and dumber--the potential waste of a useful antistaphylococcal agent: emerging fusidic acid resistance in Staphylococcus aureus. Clin Infect Dis 2006 Feb 1;42(3):394-400.
(3) Morton TM, Johnston JL, Patterson J, Archer GL. Characterization of a conjugative staphylococcal mupirocin resistance plasmid. Antimicrob Agents Chemother 1995 Jun;39(6):1272-80.
(4) Chatfield CA, O'Neill WA, Cooke RP, McGhee KJ, Issack M, Rahman M, et al. Mupirocin-resistant Staphylococcus aureus in a specialist school population. J Hosp Infect 1994 Apr;26(4):273-8.
(5) Climo MW, Ehlert K, Archer GL. Mechanism and suppression of lysostaphin resistance in oxacillin-resistant Staphylococcus aureus. Antimicrob Agents)Chemother 2001 May;45(5):1431-7.
(6) Alhanout K, Brunel JM, Raoult D, Rolain JM. In vitro antibacterial activity of aminosterols against multidrug-resistant bacteria from patients with cystic fibrosis. J Antimicrob Chemother 2009 Oct;64(4):810-4.
(7) Desbois AP, Lang S, Gemmell CG, Coote PJ. Surface disinfection properties of the combination of an antimicrobial peptide, ranalexin, with an endopeptidase, lysostaphin, against methicillin-resistant Staphylococcus aureus (MRSA). J Appl Microbiol 2009 Jul 13.
(8) Kokai-Kun JF, Walsh SM, Chanturiya T, Mond JJ. Lysostaphin cream eradicates Staphylococcus aureus nasal colonization in a cotton rat model. Antimicrob Agents Chemother 2003 May;47(5):1589-97.
(9) Loncle et al. Tetrahedron 2007, 63, 12968-12974;
(10) Salmi et al. European Journal of Medicinal Chemistry 2008, 43, 540-547;
(11) Salmi et al. Letters in Drug Design & Discovery 2008, 5, 169-172.;
(12) Salmi et al. Journal of Enzyme Inhibition and Medicinal Chemistry 2008, 23, 860-865;
(13) Loncle et al. Letters in Drug Design & Discovery 2008, 5, 388-393 ;
(14) Salmi et al. Tetrahedron 2008, 64, 4453-4459.);
(15) Daniel Amsterdam. Susceptibility testing of antimicrobials in liquid media. In Antibiotics in Laboratory Medicine. 5th Edition. Editor Victor Lorian. Lippincott Williams and Wilkins 2005. Philadelphia, USA;
(16) Banerjee R et al. In Vitro Selection and Characterization of Ceftobiprole-Resistant Methicillin-Resistant Staphylococcus aureus. Antimicrob Agents Chemother 2008, 52(6) : 2089-2096;
(17) Williams JI et al. Squalamine treatment of human tumors in nu/nu mice enhances platinum-based chemotherapies. Clin Cancer Res. 2001 Mar;7(3):724-33;
(18) LaPlante et al. In vitro activity of Lysostaphin, mupirocin, and tea tree oil against clinical methicillin-resistant Staphylococcus aureus. Diagnostic Microbiology and Infectious Disease 57 (2007) 413-418.

## Revendications

1. Composé aminostéroïdien analogue de la squalamine répondant à une formule générale comprenant un squelette de formule (I) ci-après sur lequel sont greffées 2 chaines polyaminées -NHR, R étant une chaine hydrocarbonée éventuellement substituée comportant au moins un groupe -NH₂: 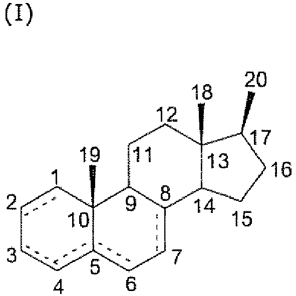 dans laquelle:
- soit toutes les liaisons doublées de traits en pointillés entre les carbones des positions 7-8, 5-6, 4-5, et 3-4 représentent une simple liaison, le squelette étant celui d'un 10, 13, 17 triméthyl cholestane,
- soit l'une des liaisons doublées de traits en pointillés entre les carbones des positions 7-8, 5-6, 4-5, et 3-4 représente une double liaison et les autres liaisons doublées de traits en pointillés sont des simples liaisons, le squelette étant celui d'un 10, 13, 17 triméthyl cholestène,
le dit composé répondant à la formule générale constituée par un dit squelette de formule (I) comportant :
a) 2 chaines identiques -NHR sur les carbones en positions 3 et, respectivement, 20, et R représente -[(CH₂)n-(NR₁)ₖ-(CH₂)ₘ]ₚ-NH₂, avec :
- n et m étant des entiers identiques ou différents de 1 à 7,
- k= 0 ou 1,
- p étant un nombre entier de 1 à 4,
- R₁ étant choisi parmi H, un alkyl en C1 à C3, un phényl et un groupe-COOalk, alk étant un alkyl en C1 à C3, R1 étant éventuellement substitué par OH, NH₂, SH, ou OSO₃H, et
b) les autres carbones du dit squelette de formule (I) comprenant un radical R₀ identique ou différent choisi parmi H, NH₂, SH, ou R₁, de préférence R₀ étant H ou OH mais avec 1 seul R₀ étant OH.

2. Composé selon la revendication 1, **caractérisé en ce que** ledit composé répond à la formule générale constituée par un dit squelette de formule (I) comportant 2 chaines identiques -NHR dans laquelle lorsque k= 0, alors p=1, ou lorsque k= 1, alors p= 2.

3. Composé selon la revendication 1 ou 2, **caractérisé en ce que** ledit composé répond aux formules générales suivantes IIIa ou IIIb, dans lesquelles R représente-[(CH₂)ₙ-(NR₁)ₖ-(CH₂)ₘ]ₚ-NH₂, et, de préférence, R étant choisi parmi -(CH₂)ₙ-NH₂ avec n= 2 à 14, et - (CH₂)₃-NH-(CH₂)₃-NH-(CH₂)₃-NH₂:

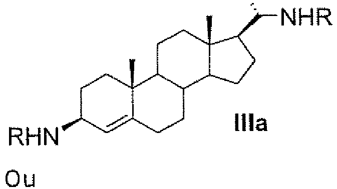

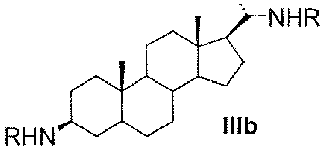

4. Composé selon la revendication 3, **caractérisé en ce que** ledit composé aminostéroïdien répond à la formule IIIa ou IIIb dans laquelle R étant choisi parmi -(CH₂)ₙ-NH₂ avec n= 12.

5. Composé selon la revendication 3, **caractérisé en ce que** ledit composé aminostéroïdien répond à la formule III-1 suivante :

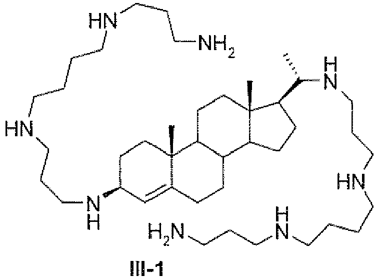

6. Composition pour une utilisation dans une méthode de traitement thérapeutique comprenant un composé selon l'une des revendications 1 à 5.

7. Composition pour une utilisation selon la revendication 6, pour une méthode de traitement thérapeutique consistant dans une décolonisation cutanéo-muqueuse de *Staphylococcus aureus.*

8. Composition pour une utilisation selon l'une des revendications 6 et 7, pour une application topique locale sur la peau ou sur une muqueuse sous forme de lotion, crème, pommade ou onguent.

9. Composition pour une utilisation selon l'une des revendications 6 à 8, ledit composé étant formulé avec un excipient lipophile.

10. Composition pour une utilisation selon l'une des revendications 6 à 9, pour une décolonisation cutanéo-muqueuse d'au moins 99,90% des bactéries *Staphylococcus aureus,* de préférence 99,99%, en pas plus de 24 heures, de préférence en pas plus de 1 heure, après seulement une seule application, ladite décolonisation étant maintenue pendant au moins 48 heures.

11. Composition pour une utilisation selon l'une des revendications 6 à 10 pour un traitement préventif ou prophylactique préopératoire visant à prévenir une infection postopératoire à *Staphylococcus aureus* chez un patient porteur sain ou asymptomatique auquel on va faire subir une opération chirurgicale.

12. Composition pour une utilisation selon l'une des revendications 6 à 11, **caractérisée en ce que** ladite application se fait sur une muqueuse nasale ou sur la peau.

13. Composition pour une utilisation selon l'une des revendications 6 à 12, **caractérisée en ce qu'**elle comporte une concentration de 0,5 à 5%, de préférence d'au moins 1% en poids de dit composé aminostéroidien analogue de la squalamine.

14. Composition pour une utilisation selon l'une des revendications 6 à 13, **caractérisée en ce que** ledit excipient lipophile est choisi parmi la vaseline, lanoline, les esters de glycérol, macrogols, esters d'acides gras et de macrogols.

15. Composition pour une utilisation selon l'une des revendications 6 à 14, **caractérisée en ce que** ledit composé aminostéroidien analogue de la squalamine antibactérien est sous forme de sel hydrosoluble, de préférence sous la forme de sel de chlorhydrate, bromhydrate, triflate, phosphate, lactate, ou succinate.

## Patentansprüche

1. Squalamin-analoge Aminosteroid-Verbindung, entsprechend einer allgemeinen Formel mit einem Gerüst nachstehender Formel (I), auf das zwei -NHR-Polyaminketten gepfropft sind, wobei R eine eventuell substituierte Kohlenwasserstoffkette mit wenigstens einer-NH₂-Gruppe ist: 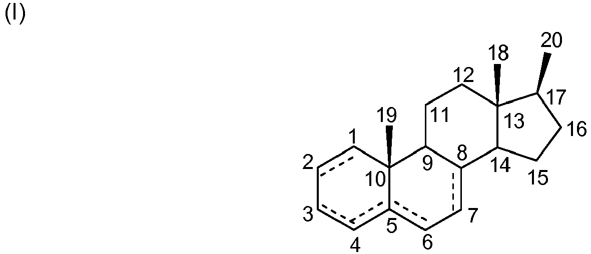 bei der:
- entweder alle mit punktierten Linien verdoppelten Bindungen zwischen den Kohlenstoffen der Positionen 7-8, 5-6, 4-5 und 3-4 eine Einfachbindung darstellen, wobei das Gerüst das eines 10, 13, 17-Trimethyl-cholestans ist,
- oder eine der mit punktierten Linien verdoppelten Bindungen zwischen den Kohlenstoffen der Positionen 7-8, 5-6, 4-5 und 3-4 eine Doppelbindung darstellt und die anderen mit punktierten Linien verdoppelten Bindungen Einfachbindungen sind, wobei das Gerüst das eines 10, 13, 17-Trimethyl-cholestens ist,
wobei die Verbindung der allgemeinen Formel bestehend aus einem Gerüst von Formel (I) entspricht, umfassend:
a) zwei identische -NHR-Ketten an den Kohlenstoffen an den Positionen 3 bzw. 20, und R stellt -[(CH₂)n-(NR₁)ₖ-(CH₂)ₘ]ₚ-NH₂ dar, wobei:
- n und m identische oder unterschiedliche ganze Zahlen von 1 bis 7 sind,
- k = 0 oder 1,
- p eine ganze Zahl von 1 bis 4 ist,
- R₁ ausgewählt ist aus H, einem C1-C3-Alkyl, einem Phenyl und einer-COOalk-Gruppe, wobei alk ein C1-C3-Alkyl ist, wobei R1 eventuell durch OH, NH₂, SH, oder OSO₃H substituiert ist, und
b) wobei die anderen Kohlenstoffe des Gerüstes von Formel (I) ein identisches oder verschiedenes Radikal R₀, ausgewählt aus H, NH₂, SH, oder R₁ umfassen, wobei vorzugsweise R₀ H oder OH ist, aber bei einem einzigen R₀ OH ist.

2. Verbindung nach Anspruch 1, **dadurch gekennzeichnet, dass** die Verbindung der allgemeinen Formel bestehend aus einem Gerüst von Formel (I) mit zwei identischen -NHR-Ketten entspricht, wobei dann, wenn k = 0, p = 1 ist oder dann, wenn k = 1, p = 2 ist.

3. Verbindung nach Anspruch 1 oder 2, **dadurch gekennzeichnet, dass** die Verbindung den folgenden allgemeinen Formeln IIIa oder IIIb entspricht, bei denen R -[(CH₂)ₙ-(NR₁)ₖ-(CH₂)ₘ]ₚ-NH₂ darstellt, und wobei vorzugsweise R ausgewählt ist aus -(CH₂)ₙ-NH₂, mit n = 2 bis 14, und -(CH₂)₃-NH-(CH₂)₃-NH-(CH₂)₃-NH₂:

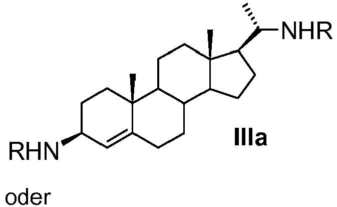

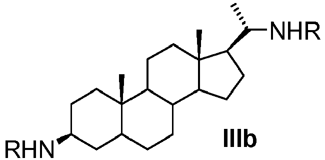

4. Verbindung nach Anspruch 3, **dadurch gekennzeichnet, dass** die Aminosteroid-Verbindung der Formel IIIa oder IIIb entspricht, bei der R ausgewählt ist aus -(CH₂)ₙ-NH₂, mit n = 12.

5. Verbindung nach Anspruch 3, **dadurch gekennzeichnet, dass** die Aminosteroid-Verbindung der folgenden Formel III-1 entspricht:

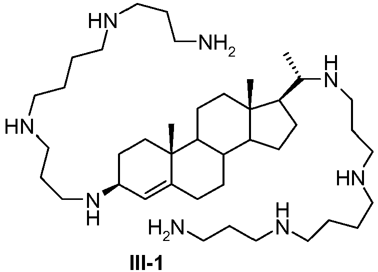

6. Zusammensetzung für eine Verwendung bei einem therapeutischen Behandlungsverfahren, umfassend eine Verbindung nach einem der Ansprüche 1 bis 5.

7. Zusammensetzung für eine Verwendung nach Anspruch 6, für ein therapeutisches Behandlungsverfahren, das in einer mukokutanen Dekolonisierung von *Staphylococcus aureus besteht.*

8. Zusammensetzung für eine Verwendung nach einem der Ansprüche 6 und 7, für eine lokale topische Anwendung auf der Haut oder auf einer Schleimhaut, in Form einer Lotion, Creme, Pomade oder Salbe.

9. Zusammensetzung für eine Verwendung nach einem der Ansprüche 6 bis 8, wobei die Verbindung mit einem lipophilen Hilfsstoff formuliert ist.

10. Zusammensetzung für eine Verwendung nach einem der Ansprüche 6 bis 9, für eine mukokutane Dekolonisierung von wenigstens 99,90 % der *Staphylococcus* aureus-Bakterien, vorzugsweise 99,99 %, in nicht mehr als 24 Stunden, vorzugsweise in nicht mehr als 1 Stunde, nach nur einer einzigen Anwendung, wobei die Dekolonisierung für wenigstens 48 Stunden aufrechterhalten wird.

11. Zusammensetzung für eine Verwendung nach einem der Ansprüche 6 bis 10 für eine präventive oder prophylaktische präoperative Behandlung, die darauf abzielt, einer postoperativen *Staphylococcus* aureus-Infektion bei einem gesunden oder asymptomatischen Trägerpatienten, der einer chirurgischen Operation unterzogen wird, vorzubeugen.

12. Zusammensetzung für eine Verwendung nach einem der Ansprüche 6 bis 11, **dadurch gekennzeichnet, dass** die Anwendung auf einer Nasenschleimhaut oder auf der Haut erfolgt.

13. Zusammensetzung für eine Verwendung nach einem der Ansprüche 6 bis 12, **dadurch gekennzeichnet, dass** sie eine Konzentration von 0,5 bis 5, vorzugsweise von wenigstens 1 Gew.-% der Squalamin-analogen Aminosteroid-Verbindung aufweist.

14. Zusammensetzung für eine Verwendung nach einem der Ansprüche 6 bis 13, **dadurch gekennzeichnet, dass** der lipophile Hilfsstoff aus Vaseline, Lanolin, Glycerinestern, Macrogolen, Fettsäure- und Macrogolestern ausgewählt ist.

15. Zusammensetzung für eine Verwendung nach einem der Ansprüche 6 bis 14, **dadurch gekennzeichnet, dass** die antibakterielle Squalamin-analoge Aminosteroid-Verbindung in Form eines wasserlöslichen Salzes, vorzugsweise in Form eines Salzes von Hydrochlorid, Hydrobromid, Triflat, Phosphat, Lactat oder Succinat vorliegt.

## Claims

1. A squalamine-analogue aminosteroid compound having a general formula comprising a backbone of formula (I) below, onto which 2 polyamine -NHR chains are grafted, R being an optionally substituted hydrocarbon chain containing at least one -NH₂ group: 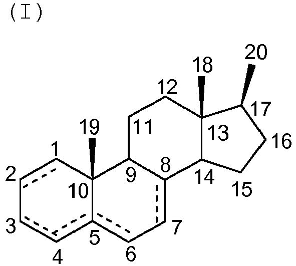 wherein:
- either all the bonds lined with a dotted line between the carbons at positions 7-8, 5-6, 4-5, and 3-4 represent a single bond, the backbone being that of a 10, 13, 17 trimethyl cholestane,
- or one of the bonds lined with a dotted line between the carbons at positions 7-8, 5-6, 4-5, and 3-4 represents a double bond and the other bonds lined with a dotted line are single bonds, the backbone being that of a 10, 13, 17 trimethyl cholestene,
said compound having the general formula constituted by a said backbone of formula (I) containing:
a) 2 identical -NHR chains on the carbons at positions 3 and, respectively, 20, and R represents -[(CH₂)ₙ-(NR₁)ₖ-(CH₂)ₘ]ₚ-NH₂, with:
- n and m, which may be identical or different, being integers from 1 to 7,
- k = 0 or 1,
- p being an integer from 1 to 4,
- R₁ being selected from H, a C1 to C3 alkyl, a phenyl and a -COOalk group, alk being a C1 to C3 alkyl, R1 being optionally substituted by OH, NH₂, SH, or OSO₃H, and
b) the other carbons of said backbone of formula (I) comprising a radical R₀, which may be identical or different, selected from H, NH₂, SH, or R₁, preferably R₀ being H or OH but with only 1 R₀ being OH.

2. The compound according to claim 1, **characterized in that** said compound has the general formula constituted by a said backbone of formula (I) containing 2 identical -NHR chains wherein when k = 0, then p = 1, or when k = 1, then p = 2.

3. The compound according to claim 1 or 2, **characterized in that** said compound has the following general formulae IIIa or IIIb, wherein R represents -[(CH₂)ₙ-(NR₁)ₖ-(CH₂)ₘ]ₚ-NH₂, and, preferably, R being selected from -(CH₂)ₙ-NH₂ with n = 2 to 14, and - (CH₂)₃-NH-(CH₂)₃-NH-(CH₂)₃-NH₂:

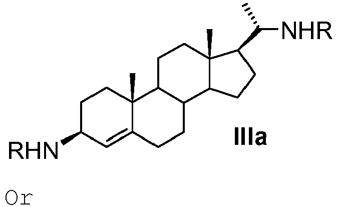

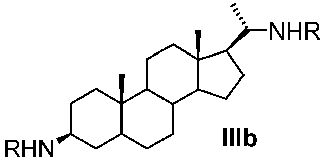

4. The compound according to claim 3, **characterized in that** said aminosteroid compound has the formula IIIa or IIIb wherein R is selected from -(CH₂)ₙ-NH₂ with n = 12.

5. The compound according to claim 3, **characterized in that** said aminosteroid compound has the following formula III-1:

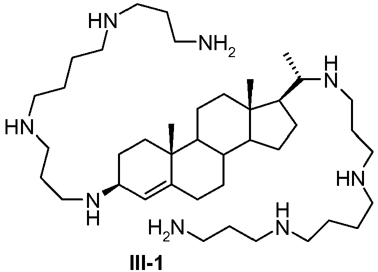

6. A composition for use in a therapeutic treatment method comprising a compound according to one of claims 1 to 5.

7. The composition for use according to claim 6, for a therapeutic treatment method consisting in mucocutaneous decolonization of *Staphylococcus aureus.*

8. The composition for use according to one of claims 6 and 7, for local topical application to the skin or to a mucosa in the form of a lotion, cream, ointment or salve.

9. The composition for use according to one of claims 6 to 8, said compound being formulated with a lipophilic excipient.

10. The composition for use according to one of claims 6 to 9, for mucocutaneous decolonization of at least 99.90% of *Staphylococcus aureus* bacteria, preferably 99.99%, in not more than 24 hours, preferably not more than 1 hour, after only one application, said decolonization being maintained for at least 48 hours.

11. The composition for use according to one of claims 6 to 10 for preoperative preventive or prophylactic treatment aimed at preventing postoperative *Staphylococcus aureus* infection in a healthy or asymptomatic carrier patient who is to undergo surgery.

12. The composition for use according to one of claims 6 to 11, **characterized in that** said application is made to a nasal mucosa or to the skin.

13. The composition for use according to one of claims 6 to 12, **characterized in that** it comprises a concentration of 0.5 to 5%, preferably at least 1%, by weight of said squalamine-analogue aminosteroid compound.

14. The composition for use according to one of claims 6 to 13, **characterized in that** said lipophilic excipient is selected from petroleum jelly, lanolin, esters of glycerol, macrogols, esters of fatty acids and of macrogols.

15. The composition for use according to one of claims 6 to 14, **characterized in that** said antibacterial squalamine-analogue aminosteroid compound is in the form of a watersoluble salt, preferably in the form of hydrochloride, hydrobromide, triflate, phosphate, lactate, or succinate salt.
